# EUROPEAN PATENT SPECIFICATION

(11) **EP 1 150 612 B1**
(45) Date of publication and mention of the grant of the patent: **11.03.2009**
(21) Application number: 00907020.2
(22) Date of filing: 25.01.2000
(51) Int. Cl.: A61B 17/22

(54) **FLEXIBLE-AGITATOR SYSTEM**
SYSTEM FÜR EINEN FLEXIBLEN AGITATOR
SYSTEME D'AGITATEUR FLEXIBLE

(30) Priority: 02.02.1999 US 241802; 04.02.1999 US 118611 P; 03.09.1999 US 389712
(43) Date of publication of application: 07.11.2001
(73) Proprietor: Shiber, Samuel, Manchester, NH 03104 (US)
(72) Inventor: Shiber, Samuel, Manchester, NH 03104 (US)
(74) Representative: Ebner von Eschenbach, Jennifer
(86) International application number: PCT/US2000/001797
(87) International publication number: WO 2000/045716

(56) References cited:
- US-A- 5 318 576
- US-A- 5 779 721
- US-A- 5 843 103

## Description

### BACKGROUND AND SUMMARY OF THE INVENTION

The rotary flexible-agitator system is designed for removing an obstruction from within a patient's vessel through a relatively small diameter tube and particularly for opening vessels, such as dialysis access grafts, that tend to become obstructed by thrombus.

An example of a present system for removing an obstruction from within a patient's vessel is disclosed in U.S. 5,318,576. That patent discloses an ellipsoidal cutter that is self-centering. The cutter has to be expanded to contact the artery's wall by pulling on a modified guidewire. A control is disclosed to pull the modified guidewire to adjust the cutter. The cutter has an effective diameter and a cross sectional diameter that are inherently the same.

Another example of a system for removing an obstruction from within a patient's vessel is disclosed in U.S. 5,843,103. This patent discloses a system wherein the rotating member is a thin hollow tubular member that rotates over a shaping member that assumes a spiral shape in the artery. The rotating member is a thin-walled tube.

Current treatments such as pharmacological, surgical or trans-catheter procedures can be time consuming, traumatic and expensive. Thus objects of the present invention are to simplify, improve and shorten the process by breaking the obstruction lodged in the vessel to pieces, and simultaneously extracting the pieces is out of the vessel. These and other objects of the invention will become apparent from the following discussion and the accompanying drawings.

### BRIEF DESCRIPTION OF THE FIGURES

FIG. 1 shows a cross-sectional view of a first embodiment of a rotary flexible-agitator system (the midsection of this embodiment and the midsections of the other embodiments are omitted to better fit on the drawing sheet.)
FIG. 1A shows a perimeter of a cross-section of a distal-agitator as viewed on the plane 3-3 marked on Figures 1,3, and 3B.
FIG. 1B shows a cross-sectional view of a first embodiment as viewed on a plane 4-4 marked on FIG. 1.
FIG. 2 shows a perspective view of a flat model that is used to explain how the relative motion between a rotary flexible agitator-shaft and a flexible-tube enables the suction to move obstruction pieces through the flexible-tube.
FIG. 3 shows a cross-sectional view of a second embodiment of a rotary flexible-agitator system.
FIG. 3A shows a cross-sectional view of a third embodiment of a rotary flexible-agitator system that utilizes an agitator-shaft and a distal-agitator that are made from a continuous flattened spiraled wire.
FIG 3B shows an enlarged view of the distal portion of the agitator-shaft, that is used in the third embodiment, that is made from a flattened wire wound on its edge and a distal-agitator that is made from the continuation of the flattened wire that was twisted a quarter of a turn and wound on its flat side.
FIG. 4 shows a cross-sectional view of a fourth embodiment of a rotary flexible-agitator system that is designed to operate over a guidewire.
FIG. 5 shows a tubular-housing inserted in a U-shaped hemodialysis access graft (shown with its ends severed.)
FIG. 6 shows a rotary flexible-agitator system inserted in a U-shaped hemodialysis access graft (shown with its ends severed.)
FIG. 7 shows a rotary flexible-agitator system, inserted in a hemodialysis access graft through an introducer. The introducer has a side-arm through which fluid can be delivered into the graft.

### DETAILED DESCRIPTION OF THE DRAWINGS

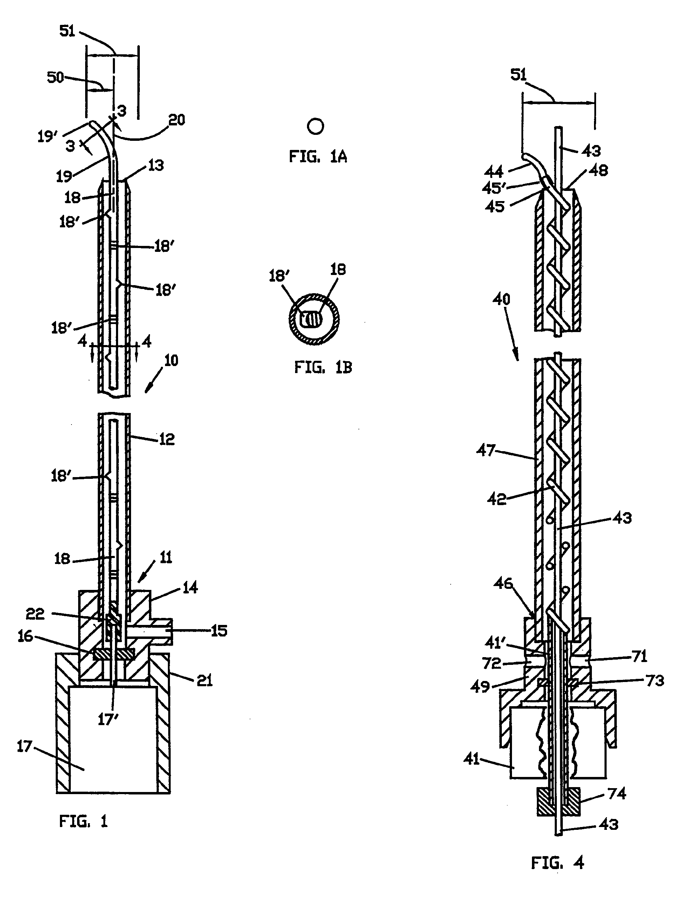

FIG. 1 shows a rotary flexible-agitator system 10 for removing an obstruction (e.g., thrombus) from within a patient's vessel. The system is comprised of a tubular-housing 11 (similar parts shall be denoted by the same numerals throughout the FIGURES) made of a flexible-tube 12 with an open distal end 13 and a proximal end 14 ("distal end" referring to the end that goes further into the vessel and "proximal end" referring to the other end). The proximal end of the tubular-housing defines a suction port 15 that is connected to the proximal end of the flexible-tube 12 (the term suction port, as used in this application, means a port in which the pressure is lower than the pressure in the vessel, and suction denotes the application of a pressure lower than the pressure in the vessel to remove the obstruction pieces from the vessel through the system.). A seal 16 is seated in the proximal end of the tubular-housing.

A motor 17 (e.g., an electric motor, an air-driven turbine or other suitable drive) is conventionally connectable to an appropriate power source (e.g., a battery or a compressed air supply, respectively, or other suitable power source. The connection to the power source and the power source are not shown.) The motor has an output shaft 17' that fits in and couples to a hollow proximal end 22 of a flexible rotary agitator 18.

The rotary flexible agitator-shaft is disposed in the tubular-housing and is preferably made from a single piece of plastic with a curved, flexible offset distal-agitator 19 that extends out of the open distal end 13 of the tubular-housing. The curved shape of the offset distal-agitator offsets its rounded end 19' away from the axis 20 of the tube 12 by a distance 50. The cross section of the offset distal-agitator, whose perimeter is illustrated in FIG. 1A, is sufficiently small and the offset distal-agitator is sufficiently flexible so that it can be inserted through the tube 12 and through a small entry puncture wound in the vessel.

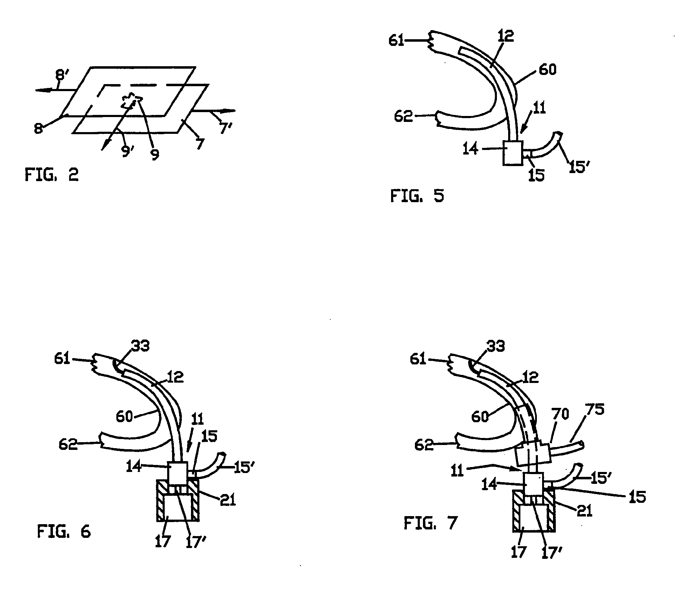

While the offset distal-agitator rotates, its effective diameter 51 (i.e., the nominal diameter within which it engages the obstruction material) is substantially larger than the cross sectional diameter of the offset distal-agitator or the diameter of the tube 12. Thus the effective diameter of the agitator is substantially larger than the smallest opening through which the agitator can be inserted. This in turn allows the size of the puncture wound that is required for insertion of the system into the vessel (note FIGURES 5 and 6) and the associated trauma to the vessel to be reduced.

A sleeve 21 fits over and aligns the motor 17 with the proximal end 14 and the flexible-tube 12.

As the offset distal-agitator breaks the obstruction in the vessel, the pieces are drawn into the open distal end 13 by suction applied to the proximal end of the flexible-tube 12 through the port 15. As the pieces enter the flexible-tube 12 the relative rotational motion between the rotary flexible agitator-shaft and the flexible-tube reduces the longitudinal friction inhibiting the motion of the pieces in the flexible-tube 12, as explained hereinafter in connection with FIG. 2. This reduction of the longitudinal friction assists the suction in moving the obstruction pieces through the flexible-tube 12. As the obstruction pieces move through the flexible-tube 12 the relative rotational motion tends to further breakdown the pieces with protrusions 18', making it easier for the suction to move them through the tubular-housing 11.

FIG. 2 shows a perspective view of a flat model that is used to explain how the relative rotational motion between the agitator-shaft and the tube is used to reduce the resistance and assist in the movement of obstruction pieces through the tube.

The model is comprised of a first plane 7 moving in a direction denoted by an arrow 7', a second plane 8 moving in an opposite direction denoted by an arrow 8' and a piece of obstruction 9 that is sandwiched between and frictionally engaged with the planes. Due to these motions full frictional forces develop between the piece 9 and the planes in directions parallel to the arrows 7' and 8'. If at the same time, even a small additional force is exerted on the piece 9 in a direction 9' (that is perpendicular to arrows 7' and 8') it will change the direction of the overall resultant force acting on the piece and cause its movement in the direction 9'. Absent the above mentioned relative movement between the planes 7 and 8, the force that would have to be exerted on the piece 9 in order to move it in the direction of arrow 9', would have to be larger than the full frictional force that can develop between the piece 9 and both planes 7 and 8. If plane 7 is envisioned as the outside surface of the agitator 18 and plane 8 is envisioned as the inside surface of the flexible tube 12 and the small force in the 9' direction is envisioned to be the force that suction exerts on the obstruction pieces, it can be understood how the relative rotational motion between the flexible-agitator 18 and the flexible-tube 12 assists the suction applied to port 15 to longitudinally move the obstruction pieces through the flexible-tube 12.

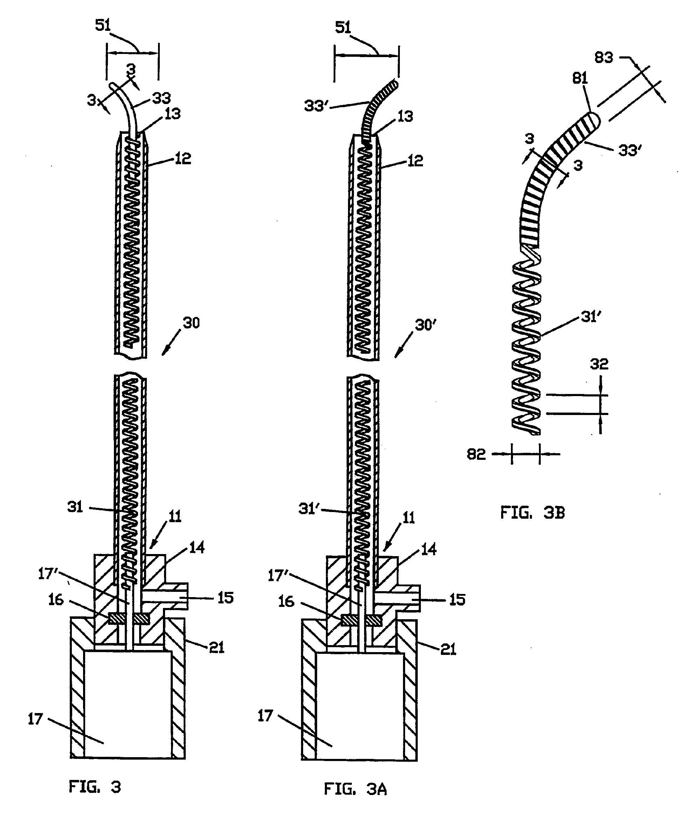

FIG. 3 shows a cross-sectional view of a second embodiment of a rotary flexible-agitator system 30 wherein the rotary flexible-agitator comprises a spiraled wire 31 (preferably a wire made of metal, e.g. stainless steel, Nitinol or other suitable alloy) that is disposed in the tubular-housing 11. The spiraled wire is preferably core-less (i.e., it has no central member) except at its two ends. A few of the spiral's proximal coils closely fit over the shaft 17' and are coupled to it, and a few of the spiral's distal coils closely fit over an offset distal-agitator 33, that is preferably made of plastic, and are coupled to it. As in the other embodiment, the effective diameter of the offset distal-agitator is indicated by numeral 51, and it is substantially larger than the opening of the tube 13 through which both the spiral 31 and the offset distal-agitator 33 are inserted.

In addition to agitating the obstruction pieces inside the flexible-tube 12, the midsection of the spiral serves as a flexible shaft for transmitting rotation and torque from the motor 17 to the distal-agitator 33. The linear length of the wire forming the spiral 31 is substantially greater than that of a non-spiraled shaft. The increased length of wire increases the flexibility of the agitator-shaft and it also increases its diameter, as compared with a non-spiraled shaft that would be flexible enough to operate in a curved vessel, as for example, is illustrated in FIGURES 6 and 7. The increased flexibility of the agitator-shaft 31 reduces the stresses that develop in the spiraled wire as well as the side forces that develop between the agitator-shaft and the flexible-tube 12 and/or the graft. The increased diameter enables the agitator-shaft to radially support the flexible-tube 12 from the inside. Without such support the flexible-tube could diametrically collapse when it is operated in a curved vessel.

FIG. 3A shows a cross-sectional view of a third embodiment of a rotary flexible-agitator system 30', wherein the agitator-shaft 31' and the offset distal-agitator 33' are made from one continuous flattened spiraled wire (the narrower side of the flattened wire's cross-section will be referred to as an "edge" and the wider side as a "flat side"). The portion of the wire that makes up the agitator-shaft is wound on its edge as illustrated in FIG 3B. This further increases the flexibility and torque carrying capacity of the agitator-shaft (as compared to a spiral wound from a round wire with same cross sectional area). The portion of the wire that makes up the distal-agitator is wound on its flat side. This reduces the gap between the coils and the likelihood of material being caught between them and it also lends itself to manufacturing the agitator-shaft with a cross-sectional diameter 82 that is larger than the offset distal-agitator cross-sectional diameter 83 which in turn leaves more of the distal end 13 open for the pieces to enter the flexible-tube 12. The distal tip 81 of the distal agitator is rounded (note FIG 3B) to reduce the trauma that it can cause to the vessel.

The motor's shaft 17' is preferably rotated in a direction so that the relative motion between the spiral and the flexible-tube 12 mechanically assists the suction applied at port 15 in conveying the obstruction material into the open distal end 13 and through the flexible-tube 12. However, since the flow through the tube is not synchronized with the rate of advancement of the spiral coils (which is the product of the spiral's rotational speed multiplied by the spiral's pitch 32), the relative motion between the rotating spiral and the flexible-tube 12 further breaks the pieces as they pass through the flexible-tube 12.

When the motor's direction of rotation is reversed the mechanical pumping action of the spiral is also reversed and opposes the suction in moving the obstruction material through the flexible-tube 12. However, by increasing the spacing between the spiral and the wall of the flexible-tube 12, or by changing the pitch of the spiral, the mechanical pumping action of the spiral can be adjusted so that the suction applied at the port 15 will dominate the direction of the flow through the flexible-tube 12. While the reversal of the mechanical pumping action reduces the rate of flow through the flexible-tube 12, it increases the opportunity of the obstruction pieces to collide with the rotating spiral and be further broken down. Optionally, the motor can be periodically, manually or automatically, reversed to assist in releasing pieces of the obstruction that may have become stuck in the flexible-tube 12 or wrapped around the agitator. (This technique is applicable to any of the embodiments.)

As in the other embodiments, the relative rotational motion between the flexible-agitator and the flexible-tube 12 assists the suction applied to port 15 to longitudinally move the obstruction pieces through the flexible-tube.

FIG. 4 shows a cross-sectional view of a fourth embodiment of a rotary flexible-agitator system 40. In this embodiment the shaft 41' of the motor 41 is a tube, that together with a spiral 42, which is affixed and coupled to its distal end, define a continuous passageway that can accommodate a guidewire 43 or similar elongated object (e.g., an optic fiber or an ultrasound probe). The spiral 42 is also preferably made of a metal wire and its distal end 45 is affixed to a hollow end 45' of an offset distal-agitator 44.

The fourth embodiment's tubular-housing 46 is made of a flexible-tube 47 with an open distal end 48 and a proximal end 49 which defines a suction port 71, that is connected to the proximal end of the flexible-tube 47. An optional second port 72 can be used to introduce fluid into the proximal end 49. Such fluid can be used to dilute the slurry of obstruction pieces that are removed through port 71. A seal 73, seated in the proximal end of the tubular-housing, minimizes leakage around the motor shaft. A seal 74, that is attached to the proximal end of the shaft 41', minimizes leakage around the guidewire 43 and through the shaft 41'. The proximal end 49 defines an open cylinder that fits around the motor 41 and concentrically aligns the motor with the flexible-tube 47.

FIG. 5 shows the tubular-housing 11 inserted in a vessel such as a U-shaped hemodialysis access graft 60 (the graft's ends, 61 and 62, are normally connected to a vein and an artery. The vein and the artery are not shown).

FIG. 6 shows a rotary flexible-agitator system where a flexible offset distal-agitator 33 is connected to a flexible agitator-shaft that is disposed (and hidden) in the tubular-housing 11 and is connected to a motor's shaft 17'.

FIG. 7 shows the rotary flexible-agitator system, inserted in a the hemodialysis access graft 60 through a commercially available introducer 70 with its own side-arm 75, through which fluid can be introduced into the graft.

Various modifications and substitutions may be made to the rotary flexible-agitator system without departing from the spirit of the invention or the scope of the claims. For example, the flexible offset distal agitator shown in the FIGURES is deemed preferable for its simplicity and ease of insertion into the vessel through a small puncture wound without it having to be collapsed. Nevertheless, the preferable agitator can be replaced with, for example, a balloon-shape distal agitator distal agitator whose effective diameter is substantially larger than the smallest opening through which this distal agitator can be inserted in a collapsed position.

The process of removing an obstruction from the vessel 60 comprises the following steps:
Inserting a tubular-housing 11 into the vessel 60 as shown in FIG. 5. Tubular-housing 11 can be a commercially available introducer sheath of the type that is commonly used to gain percutaneous access to a patient's vessels or a similar structure. When such an introducer is inserted into a vessel it is usually inserted over a closely fitting dilator, which is retracted once the introducer is in the vessel, leaving the seal 16 (note FIG. 1) to seal the proximal end of the introducer. At this point, which is illustrated in FIG. 5 suction can be applied to the port 15 through a tube 15', to try and remove the obstruction. Alternatively or adjunctively, if suction alone does not sufficiently remove the obstruction, the motor driven rotary flexible-agitator, with the offset distal-agitator, can be inserted through the seal 16 until the offset distal-agitator extends out of the open distal end of the tubular-housing and the motor 17 is seated in the sleeve 21 that concentrically aligns it with the tubular-housing as illustrated in FIG. 6.

Then suction can be applied and the motor activated to rotate the rotary flexible agitator-shaft and the offset distal-agitator. As the rotating offset distal-agitator engages with and breaks the obstruction (not shown) in the vessel 60 the suction draws the pieces through the open distal end into the flexible-tube 12. It should be understood that while working in the vessel the effective diameter of the flexible distal-agitator could be altered by the internal topology of the obstructed vessel as well as by centripetal forces.

The relative motion between the rotary flexible agitator-shaft and the tubular-housing 11 reduces the longitudinal frictional forces (frictional forces that oppose the advancement of obstruction pieces through the flexible-tube 12), allowing the pieces to be suctioned through the tube. In addition, the relative motion between the flexible-tube 12 and the radial protrusions 18' of the rotary flexible agitator-shaft shown in FIG. 1 or of the spiral coils shown in FIG. 3 further break the pieces as they pass through the tubular-housing 11.

When the fourth embodiment that is shown in FIG. 4 is used, a preparatory step of inserting a guide wire (as previously mentioned, similarly shaped elongated items such as optical fibers or ultrasonic transducers can also serve as guidewires) is added. Then the embodiment of FIG. 4 can be inserted and directed over the guidewire to break down the obstruction and suction the pieces through the tubular-housing 46 similarly to the other embodiments.

The rotary flexible-agitator system can be inserted directly into the obstructed vessel, as shown in FIG. 6, or it can be inserted through an introducer, as shown in FIG. 7. The introducer is preferably equipped with a side arm through which fluid (e.g., saline solution containing heparin and radio-opaque contrast material to prevent reoccurrence of thrombi and assist in fluoroscopically imaging the process, respectively) can be introduced into the vessel.

It should be understood that the method described herein does not form part of the invention but represents background art that is useful for understanding the invention.

It should be understood that the number and sequence of steps can be modified within the scope of the claims.

## Claims

1. A rotary agitator system for removing an obstruction from within a patient's vessel comprising in combination:
a tubular-housing (11) having a flexible-tube (12) with an open distal end (13) and a suction port (15) that is connected to said flexible-tube (12), and
a motor driven flexible agitator-shaft (17') disposed in the tubular-housing (11) **characterized in that** the motor driven flexible agitator-shaft (17') has a flexible offset distal-agitator (19) that extends out of the open distal end (13) of the flexible-tube (12), and an effective diameter (51) of the offset distal-agitator (19) being substantially larger than its cross sectional diameter (83).

2. A rotary agitator system as in claim 1 wherein the agitator-shaft (17') comprises a spiraled wire (31).

3. A rotary agitator system as in claim 2 wherein the spiraled wire (31) radially supports the flexible-tube (12) while the rotary agitator system is operated in a curved vessel.

4. A rotary agitator system and in claim 1 wherein the agitator-shaft (17') and the offset distal-agitator (19) are made from a continuous spiraled wire (31).

5. A rotary agitator system as in claim 4 wherein the agitator-shaft (17') is made of a flattened wire wound on its edge and the distal-agitator (19) is made from the flattened wire wound on its side.

6. A rotary agitator system as in claim 4 wherein the spiraled wire (31) is core-less.

7. A rotary agitator system as in claim 4 wherein the cross-sectional diameter (82) of the agitator shaft (17') is larger than the cross-sectional diameter (83) of the offset distal-agitator (19).

8. A rotary agitator system as in claim 1 wherein the distal end of the flexible-tube (12) is curved.

9. A rotary agitator system as in claim 1 wherein a distal end (13) of the agitator-shaft (17') is coupled to a flexible offset distal-agitator (19) that is made of a plastic material.

10. A rotary agitator system as in claim 1 wherein the rotary flexible agitator-shaft (17') has radial protrusions (18) that, during the relative motion between the rotary flexible agitator-shaft (19) and the flexible-tube (12), further break the pieces as they pass through the flexible-tube (12).

## Patentansprüche

1. Drehbares Agitatorsystem zum Entfernen eines Hindernisses aus einem Gefäß eines Patienten, wobei das System in Kombination folgendes umfasst:
ein röhrenförmiges Gehäuse (11) mit einem elastischen Schlauch (12) mit einem offenen distalen Ende (13) und einem Sauganschluss (15), der mit dem genannten elastischen Schlauch (12) verbunden ist; und
eine motorisch angetriebene, flexible Agitatorwelle (17'), die in dem röhrenförmigen Gehäuse (11) angeordnet ist, **dadurch gekennzeichnet, dass** die motorisch angetriebene, flexible Agitatorwelle (17') einen flexiblen, versetzten distalen Agitator (19) aufweist, der sich von dem offenen distalen Ende (13) des flexiblen Schlauchs (12) erstreckt, und wobei ein effektiver Durchmesser (51) des versetzten distalen Agitators (19) deutlich größer ist als dessen Querschnittsdurchmesser (83).

2. Drehbares Agitatorsystem nach Anspruch 1, wobei die Agitatorwelle (17') einen spiralförmigen Draht (31) umfasst.

3. Drehbares Agitatorsystem nach Anspruch 2, wobei der spiralförmige Draht (31) radial den elastischen Schlauch (12) stützt, während das drehbare Agitatorsystem in einem gekrümmten Gefäß betrieben wird.

4. Drehbares Agitatorsystem nach Anspruch 1, wobei die Agitatorwelle (17') und der versetzte distale Agitator (18) aus einem spiralförmigen Endlosdraht (31) hergestellt werden.

5. Drehbares Agitatorsystem nach Anspruch 4, wobei die Agitatorwelle (17') aus einem abgeflachten Draht, der auf dessen Kante gewickelt ist, hergestellt wird, und wobei der distale Agitator (19) aus dem abgeflachten Draht, der auf dessen Seite gewickelt ist, hergestellt wird.

6. Drehbares Agitatorsystem nach Anspruch 4, wobei der spiralförmige Draht (31) kernlos ist.

7. Drehbares Agitatorsystem nach Anspruch 4, wobei der Querschnittsdurchmesser (82) der Agitatorwelle (17') größer ist als der Querschnittsdurchmesser (83) des versetzten distalen Agitators (19).

8. Drehbares Agitatorsystem nach Anspruch 1, wobei das distale Ende des flexiblen Schlauchs (12) gekrümmt ist.

9. Drehbares Agitatorsystem nach Anspruch 1, wobei ein distales Ende (13) der Agitatorwelle (17') mit einem flexiblen, versetzten distalen Agitator (19) gekoppelt ist, der aus einem Kunststoff hergestellt wird.

10. Drehbares Agitatorsystem nach Anspruch 1, wobei die drehbare flexible Agitatorwelle (17') radiale Vorsprünge (18) aufweist, die während der relativen Bewegung zwischen der drehbaren flexiblen Agitatorwelle (19) und dem flexiblen Schlauch (12) die Elemente weiter brechen, wenn diese durch den flexiblen Schlauch (12) verlaufen.

## Revendications

1. Système d'agitateur rotatif pour retirer une obstruction d'un vaisseau d'un patient comprenant en combinaison :
un logement (11) tubulaire comportant un tube (12) flexible avec une extrémité distale (13) ouverte et un orifice d'aspiration (15) qui est relié audit tube (12) flexible, et
un arbre d'agitateur (17') flexible entraîné par un moteur disposé dans le logement (11) tubulaire, **caractérisé en ce que** l'arbre d'agitateur (17') flexible entraîné par un moteur comporte un agitateur distal (19) décalé flexible qui s'étend hors de l'extrémité distale (13) ouverte du tube (12) flexible, et un diamètre efficace (51) de l'agitateur distal (19) décalé est sensiblement supérieur à son diamètre en coupe (83).

2. Système d'agitateur rotatif selon la revendication 1, dans lequel l'arbre d'agitateur (17') comprend un câble (31) enroulé en spirale.

3. Système d'agitateur rotatif selon la revendication 2, dans lequel le câble (31) enroulé en spirale supporte radialement le tube (12) flexible alors que le système d'agitateur rotatif est utilisé dans un vaisseau incurvé.

4. Système d'agitateur rotatif selon la revendication 1, dans lequel l'arbre d'agitateur (17') et l'agitateur distal (19) décalé sont réalisés à partir d'un câble (31) enroulé en spirale continu.

5. Système d'agitateur rotatif selon la revendication 4, dans lequel l'arbre d'agitateur (17') est constitué d'un câble aplati enroulé sur son bord et l'agitateur distal (19) est réalisé à partir d'un câble aplati enroulé sur son côté.

6. Système d'agitateur rotatif selon la revendication 4, dans lequel le câble (31) enroulé en spirale est sans âme.

7. Système d'agitateur rotatif selon la revendication 4, dans lequel le diamètre en coupe (82) de l'arbre d'agitateur (17') est supérieur au diamètre en coupe (83) de l'agitateur distal (19) décalé.

8. Système d'agitateur rotatif selon la revendication 1, dans lequel l'extrémité distale du tube (12) flexible est incurvée.

9. Système d'agitateur rotatif selon la revendication 1, dans lequel une extrémité distale (13) de l'arbre d'agitateur (17') est couplée à un agitateur distal (19) décalé flexible qui est réalisé en une matière plastique.

10. Système d'agitateur rotatif selon la revendication 1, dans lequel l'arbre d'agitateur (17') flexible rotatif comporte des protubérances radiales (18) qui, pendant le mouvement relatif entre l'arbre d'agitateur (19) flexible rotatif et le tube (12) flexible, brisent en outre les morceaux alors qu'ils passent à travers le tube (12) flexible.
